# DEMANDE DE BREVET EUROPEEN

(11) **EP 1 982 675 A1**
(43) Date de publication de la demande: **22.10.2008**
(21) Numéro de dépôt: 07290510.2
(22) Date de dépôt: 19.04.2007
(51) Int. Cl.: A61F 2/34, A61F 2/46

(54) **Cupule pour implant cotyloidien.**

(71) Demandeur: Implant reduction, 91560 Crosne (FR)
(72) Inventeur: Herve, Jean-Louis, 91560 Crosne (FR); Perron, Claude-Henry, 91310 Leuville-Sur-Orges (FR); Dufour, Guillaume, 92600 Asnieres Sur Seine (FR)
(74) Mandataire: Puiroux, Guy

(57) **Abrégé**

La présente invention concerne une cupule cotyloïdienne (7) comprenant une coquille hémisphérique (3) destinée à être appliquée dans la cavité cotyloïdienne (7), et un élément de blocage (5) de ladite coquille (3) ou insert.

Cette cupule est caractérisée en ce que la partie supérieure de la coquille (3) est pourvue d'un orifice axial fileté (13), l'insert comporte à sa partie supérieure un embout fileté (16) apte à se visser dans un orifice fileté (13) de la coquille (3) de façon à faire se dilater celle-ci et assurer ainsi son blocage dans ladite cavité cotyloïdienne (7).

## Description

La présente invention concerne une cupule destinée à recevoir un implant cotyloïdien d'une prothèse fémorale et concerne plus particulièrement des moyens de fixation de cette cupule à l'intérieur de la cavité cotyloïdienne.

On sait qu'une prothèse totale de hanche se compose essentiellement d'une pièce fémorale et d'une pièce cotyloïdienne. La pièce fémorale est constituée d'une tige qui prend place dans la diaphyse fémorale et qui se termine à une extrémité par une tête sphérique. La pièce cotyloïdienne est généralement constituée d'une cupule métallique qui est fixée dans la cavité cotyloïdienne et à l'intérieur de laquelle est disposé un insert en matière de synthèse, notamment en polyéthylène, qui est creusé d'une cavité hémisphérique de façon à recevoir, à pivotement, la tête de la pièce fémorale.

La fixation de la cupule métallique à l'intérieur de la cavité cotyloïdienne peut s'effectuer de différentes façons, et notamment par des moyens de scellement à l'aide de ciments appropriés, par vissage, ou simplement par un montage à pression dans la cavité. Afin de faciliter cette fixation et empêcher le glissement ou le basculement de la cupule dans la cavité, les parois externes de celle-ci sont la plupart du temps pourvues de divers types de rainures et de crantages divers destinés à prévenir tout glissement.

Il arrive cependant lorsque des cupules de conception rigide sont mises en place dans des cavités osseuses qui, en raison de leur nature, auraient nécessité des cupules pourvues d'un certain niveau de déformabilité, qu'elles causent des douleurs au patient.

On a proposé dans le brevet FR-B-2 834 630 une cupule pour implant cotyloïdien permettant de remédier aux divers inconvénients précités, notamment au niveau de la simplification et de la facilité de mise en oeuvre ainsi qu'à celui de l'efficacité de la fixation sur le plan mécanique. Suivant l'invention objet de ce brevet, la cupule est composée essentiellement de deux coquilles, à savoir une première coquille hémisphérique qui vient prendre place dans la cavité cotyloïdienne et qui comporte un renflement sur son bord externe périphérique un renflement, le bord externe étant relié au sommet de la coquille par une zone déformable élastiquement permettant un encastrement au moins partiel du renflement périphérique dans une gorge circulaire correspondante creusée à cet effet dans la cavité cotyloïdienne, et une seconde coquille rigide indéformable venant se fixer dans la première permettant de bloquer toute déformation ultérieure de celle-ci.

La présente invention a pour but de faciliter encore à la fois la mise en place et la fixation de la cupule dans la cavité cotyloïdienne.

La présente invention a ainsi pour objet une cupule destinée à être implantée dans une cavité cotyloïdienne et destinée à la réception de la tête sphérique d'un implant fémoral, comprenant d'une part une coquille hémisphérique destinée à être appliquée dans la cavité cotyloïdienne, comportant sur son bord externe périphérique un renflement, ce bord externe étant relié au sommet de la coquille par une zone déformable élastiquement, de façon à permettre un encastrement, au moins partiel, du renflement périphérique dans une gorge circulaire correspondante creusée dans la cavité cotyloïdienne et, d'autre part, un élément de blocage de ladite coquille, caractérisée en ce que :
- la partie supérieure de la coquille est pourvue d'un orifice axial fileté,
- l'élément de blocage, ou insert, est formé d'une pièce de révolution autour d'un axe comportant à sa partie supérieure un embout fileté apte à se visser dans l'orifice fileté de la coquille, la forme globale de l'insert étant telle qu'au cours de ce vissage, elle assure la déformation de la zone déformable de la coquille, de façon à faire se dilater celle-ci et assurer ainsi son blocage dans ladite cavité cotyloïdienne, la face interne de l'insert étant creusée d'une cavité hémisphérique destinée à recevoir la tête de l'implant fémoral.

Dans un mode de mise en oeuvre de l'invention la zone de la coquille déformable élastiquement est obtenue par une série de fentes radiales réalisées sur la périphérie externe de celle-ci et qui traversent ledit renflement périphérique.

Suivant l'invention l'insert sera avantageusement pourvu d'un renflement circulaire destiné à prendre place dans le renflement périphérique de celle-ci de façon, au cours du vissage de l'insert dans la coquille, à favoriser la dilatation de ladite zone déformable de celle-ci.

Dans une variante intéressante de la présente invention, la base de l'insert sera pourvue d'une rainure circulaire destinée à recevoir un élément en mesure d'être détecté par les rayons X, tel que par exemple un fil témoin métallique.

Afin d'assurer une meilleure solidarisation en rotation de la cupule dans la cavité cotyloïdienne, la partie supérieure externe de la coquille sera avantageusement pourvue d'au moins un élément, tel que par exemple une pointe, apte à pénétrer dans le tissu cotyloïdien lors de la mise en place de cette dernière.

La présente invention s'est révélée particulièrement intéressante en ce qu'elle permet une progressivité dans la déformation de la coquille ce qui permet au praticien de contrôler de façon précise la valeur de la déformation de celle-ci en fonction de la qualité des tissus osseux dans laquelle elle prend place, créant ainsi une « dynamisation » contrôlée de ceux-ci.

Par ailleurs si l'on souhaite retirer une cupule précédemment implantée dans une cavité cotyloïdienne, il suffit au praticien de dévisser et retirer l'insert pour que la zone déformable élastiquement reprenne sa position initiale permettant ainsi le retrait de la coquille de la cavité cotyloïdienne.

On décrira ci-après à titre d'exemple non limitatif, une forme d'exécution de la présente invention, en référence au dessin annexé sur lequel :
Les figures 1 et 2 sont des vues en perspective respectivement d'une coquille et d'un insert constituant une cupule suivant l'invention,
La figure 3 est une vue en coupe axiale et diamétrale d'une coquille mise en place dans une cavité cotyloïdienne, et de l'insert qui lui est associé avant mise en place dans la coquille.
La figure 4 est une vue en coupe axiale et diamétrale de la coquille, de son insert et d'une tête d'un implant fémoral,
La figure 5 est une vue en perspective d'une variante de réalisation d'une coquille utilisée dans une cupule suivant l'invention,
La figure 6 est une vue partielle axiale d'un outil de mise en place de la cupule dans une cavité cotyloïdienne, et de l'insert qui lui est associé avant mise en place dans la coquille.
La figure 7 est une vue de dessus de l'outil représenté sur la figure 6.

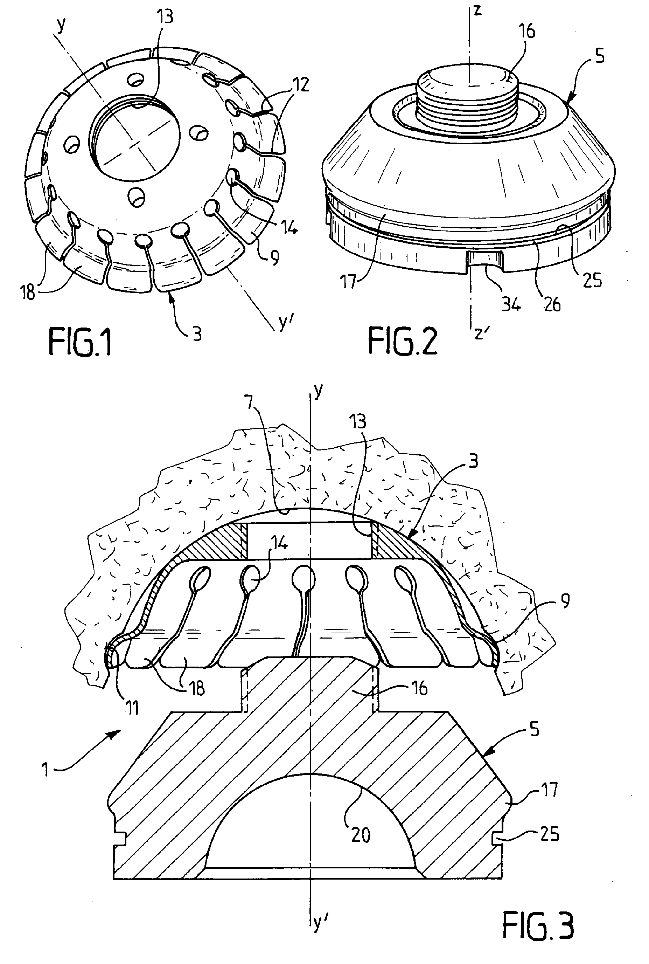

On a représenté sur les figures 1 à 4 une cupule 1 suivant l'invention qui est essentiellement constituée d'une coquille métallique 3 de forme globale hémisphérique et d'axe yy' et d'un insert 5 destiné à se loger dans celle-ci.

La coquille 3 est destinée à prendre place dans une cavité cotyloïdienne 7 qui, à cet effet, ainsi qu'il en est habituellement dans l'état antérieur de la technique, a subi un usinage destiné à parfaire sa forme sphérique. La coquille 3 qui est réalisée par exemple en métal, et notamment en tout métal agréé pour les implants médicaux, a une base qui forme un renflement 9 qui est destiné à prendre place dans une rainure circulaire 11 préalablement creusée à cet effet dans la cavité cotyloïdienne 7 au moyen d'une fraise de forme spécifique, par exemple du type de celle décrite dans le brevet FR-2.834.630 précédemment cité. Par ailleurs la partie supérieure de la coquille 3 est percée d'un trou fileté 13.

Afin de conférer à la base de la coquille 3 une certaine déformabilité, cette dernière à été découpée d'une série de seize fentes radiales 12, dans l'exemple de mise en oeuvre représenté sur le dessin, qui débouchent à une extrémité sur l'extérieur et à leur autre extrémité dans des trous 14. Le nombre, la largeur et la longueur de ces fentes 12 permettent au concepteur de contrôler l'élasticité conférée à la base de la coquille 3.

L'insert 5, dans le présent mode de mise en oeuvre de l'invention, peut être réalisé en céramique ou en matière de synthèse, par exemple en polymère haute densité (PEHD) ou en poly(aryléthercétone) (PEEK). Cet insert a la forme d'un solide de révolution autour d'un axe zz'. Il comporte à sa partie supérieure un embout fileté 16 par lequel il vient se visser à l'intérieur de la coquille 3 dans le trou fileté 13, si bien que les axes de révolution de ces deux éléments sont confondus et représentés sur les dessins par l'axe yy'. La partie inférieure de l'insert 5 comporte un bourrelet périphérique 17 qui, lors de son vissage dans la coquille 3, est amené à prendre place à l'intérieur du renflement 9 de celle-ci. La base de l'insert 5 est creusée d'une cavité hémisphérique 20 qui est destinée à recevoir la tête 22 d'une pièce fémorale 23. La forme globale de la partie supérieure de l'insert est telle que, lors de son vissage dans la coquille 3, elle déforme les languettes 18 formées entre les fentes 12, dans le sens d'une expansion, si bien que l'on assure ainsi la fixation de la cupule 1 dans la cavité cotyloïdienne 7.

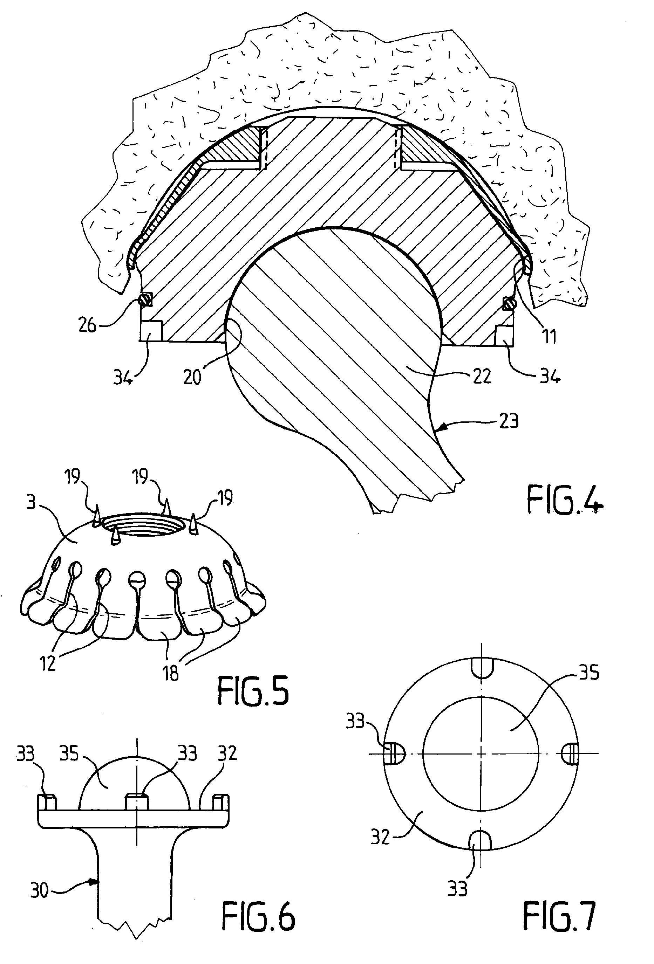

Dans un mode de mise en oeuvre particulièrement intéressant de l'invention on fera en sorte que la distance séparant le sommet de la cavité cotyloïdienne de la rainure circulaire 11 soit telle que la coquille 3 étant positionnée dans la cavité cotyloïdienne 7, il soit nécessaire de déformer légèrement les languettes 18 pour que celles-ci viennent s'encliqueter dans la rainure circulaire 11. Dans ces conditions, une fois la coquille ainsi encliquetée, elle se trouve maintenue dans la cavité cotyloïdienne et le praticien a alors tout « loisir » pour assurer sa fixation définitive en venant y visser l'insert 5. Le vissage sera facilité si l'on prévoit des moyens prévenant toute rotation de la coquille tels que par exemple, ainsi que représenté sur la figure 5, la fixation de pointes axiales 19 disposées au sommet de la coquille 3.

Un avantage important de la présente invention est que la fixation de la cupule 1 est progressive et peut en conséquences être dosée à la guise du praticien en fonction par exemple de la qualité des parties osseuses du patient.

Un autre avantage tient au fait que si, dans le temps, une nouvelle intervention s'avère nécessaire, le démontage de la cupule se fera aisément par un simple dévissage et une rétraction de l'insert.

La périphérie inférieure de l'insert 5 sera préférentiellement creusée d'une rainure 25 qui reçoit un fil métallique 26. Ce dernier, en raison de son caractère radio-opaque aux rayons X formera un témoin qui sera visible sur les radiographies et donnera au praticien, une fois l'opération terminée, des indications sur le positionnement angulaire de la cupule.

Le vissage de l'insert 5 se fera préférentiellement au moyen d'un outil spécifique 30 qui, ainsi que représenté sur les figures 6 et 7, sera constitué d'une tige, ou manche 31, dont la partie supérieure se termine par un plateau circulaire 32 sur lequel prend appui la base de l'insert 5. La partie supérieure du plateau 32 comporte d'une part quatre ergots 33 destinés à prendre place dans des encoches correspondantes 34 réalisés dans l'insert 5 et qui assurent la liaison en rotation de l'outil 30 et de celui-ci et, d'autre part, une proéminence hémisphérique 35 de même diamètre que la cavité 20 prévue dans l'insert 5 et qui est destinée à prendre place dans celle-ci, cette proéminence participant au maintien de l'insert sur l'outil.

## Revendications

1. Cupule destinée à être implantée dans une cavité cotyloïdienne (7) et destinée à la réception de la tête sphérique (22) d'un implant fémoral (23), comprenant d'une part une coquille hémisphérique (3) destinée à être appliquée dans la cavité cotyloïdienne (7), comportant sur son bord externe périphérique un renflement (9), ce bord externe étant relié au sommet de la coquille (3) par une zone déformable élastiquement, de façon à permettre un encastrement, au moins partiel, du renflement périphérique dans une gorge circulaire (11) correspondante creusée dans la cavité cotyloïdienne (7) et, d'autre part, un élément de blocage (5) de ladite coquille (3), **caractérisée en ce que** :
- la partie supérieure de la coquille (3) est pourvue d'un orifice axial fileté (13),
- l'élément de blocage, ou insert (5), est formé d'une pièce de révolution autour d'un axe (yy') comportant à sa partie supérieure un embout fileté (16) apte à se visser dans l'orifice fileté (13) de la coquille (3), la forme globale de l'insert (5) étant telle qu'au cours de ce vissage, elle assure la déformation de la zone déformable de la coquille, de façon à faire se dilater celle-ci et assurer ainsi son blocage dans ladite cavité cotyloïdienne (7), la face interne de l'insert (5) étant creusée d'une cavité hémi-sphérique (20) destinée à recevoir la tête sphérique (22) de l'implant fémoral (23).

2. Cupule suivant la revendication 1, **caractérisée en ce que** la zone déformable élastiquement de la coquille (3) est obtenue par une série de fentes radiales (12) réalisées sur la périphérie externe de celle-ci et qui traversent ledit renflement périphérique (9).

3. Cupule suivant l'une des revendications 1 ou 2, **caractérisée en ce que** l'insert (5) est pourvu d'un renflement circulaire (17) destiné à prendre place dans le renflement périphérique (9) de celle-ci de façon, au cours du vissage de l'insert (5) dans la coquille (3), à faire se dilater ladite zone déformable élastiquement.

4. Cupule suivant l'une des revendications précédentes, **caractérisée en ce que** la base de l'insert (3) est pourvue d'une rainure circulaire (25) destinée à recevoir un élément (26) en mesure d'être détecté aux rayons X, tel que par exemple un fil métallique.

5. Cupule suivant l'une des revendications précédentes **caractérisée en ce que** la partie supérieure externe de la coquille (3) est pourvue d'au moins un élément en mesure de pénétrer dans le tissu cotyloïdien lors de la mise en place de cette dernière.
